# EUROPÄISCHE PATENTSCHRIFT

(11) **EP 3 870 144 B1**
(45) Veröffentlichungstag und Bekanntmachung des Hinweises auf die Patenterteilung: **27.05.2026**
(21) Anmeldenummer: 19742277.7
(22) Anmeldetag: 01.07.2019
(51) Int. Cl.: A61K 9/20, A61K 31/155, A61K 47/14, A61K 9/00

(54) **LIPOPHILE TRANSPORTPARTIKEL FÜR KOSMETISCHE ODER PHARMAZEUTISCHE WIRKSTOFFE**
LIPOPHILIC TRANSPORT PARTICLES FOR COSMETIC OR PHARMACEUTICAL ACTIVE INGREDIENTS
PARTICULES DE TRANSPORT LIPOPHILE POUR PRINCIPES ACTIFS COSMÉTIQUES OU PHARMACEUTIQUES

(30) Priorität: 22.10.2018 WO PCT/DE2018/000302; 11.12.2018 WO PCT/DE2018/000363; 30.04.2019 WO PCT/DE2019/000115
(43) Veröffentlichungstag der Anmeldung: 01.09.2021
(73) Patentinhaber: IOI Oleo GmbH, 20459 Hamburg (DE)
(72) Erfinder: LOCHMANN, Dirk, 58730 Fröndenberg (DE); REYER, Sebastian, 45149 Essen (DE); SALAR BEHZADI, Sharareh, 1080 Wien (AT); STEHR, Michael, 45881 Gelsenkirchen (DE); ZIMMER, Andreas, 8010 Graz (AT)
(74) Vertreter: Kayser, Christoph
(86) Internationale Anmeldenummer: PCT/DE2019/000171
(87) Internationale Veröffentlichungsnummer: WO 2020/083413

(56) Entgegenhaltungen:
- EP-A1- 0 514 008
- EP-A2- 0 368 247
- WO-A1-2011/110926
- WO-A2-2013/183062
- US-A- 4 755 387
- US-A- 5 891 476
- US-A1- 2010 004 473
- AKIYAMA Y ET AL: "Novel oral controlled-release microspheres using polyglycerol esters of fatty acids", JOURNAL OF CONTROLLED RELEASE, ELSEVIER, AMSTERDAM, NL, vol. 26, no. 1, 1 July 1993 (1993-07-01), pages 1 - 10, XP023744544, ISSN: 0168-3659, [retrieved on 19930701], DOI: 10.1016/0168-3659(93)90203-H
- BECKER KARIN ET AL: "Solvent-Free Melting Techniques for the Preparation of Lipid-Based Solid Oral Formulations", PHARMACEUTICAL RESEARCH, SPRINGER NEW YORK LLC, US, vol. 32, no. 5, 19 March 2015 (2015-03-19), pages 1519 - 1545, XP035444577, ISSN: 0724-8741, [retrieved on 20150319], DOI: 10.1007/S11095-015-1661-Y
- AOSHIMA H ET AL: "Glycerin fatty acid esters as a new lubricant of tablets", INTERNATIONAL JOURNAL OF PHARMACEUTICS, ELSEVIER, NL, vol. 293, no. 1-2, 11 April 2005 (2005-04-11), pages 25 - 34, XP027624098, ISSN: 0378-5173, [retrieved on 20050411]
- JASPART S ET AL: "SOLID LIPID MICROPARTICLES: FORMULATION, PREPARATION, CHARACTERISATION, DRUG RELEASE AND APPLICATIONS", EXPERT OPINION ON DRUG DELIVERY, INFORMA HEALTHCARE, GB, vol. 2, no. 1, 1 January 2005 (2005-01-01), pages 75 - 87, XP001206211, ISSN: 1742-5247, DOI: 10.1517/17425247.2.1.75
- LUIS FELIPE COSTA SILVA ET AL: "Preparation and characterization of quercetin-loaded solid lipid microparticles for pulmonary delivery", POWDER TECHNOLOGY - ELECTROSTATIC PHENOMENA IN PARTICULATE PROCESSES, vol. 239, 1 February 2013 (2013-02-01), Basel (CH), pages 183 - 192, XP055636805, ISSN: 0032-5910, DOI: 10.1016/j.powtec.2013.01.037
- VANNA SANNA ET AL: "Preparation and in vivo toxicity study of solid lipid microparticles as carrier for pulmonary administration", AAPS PHARMSCITECH, vol. 5, no. 2, 1 June 2004 (2004-06-01), pages 17 - 23, XP055636826, DOI: 10.1208/pt050227
- YOHKO A ET AL: "Mechanism of drug release from polyglycerol ester of fatty acid-based microspheres", JOURNAL OF CONTROLLED RELEASE, ELSEVIER, AMSTERDAM, NL, vol. 27, no. 1, 1 October 1993 (1993-10-01), pages 37 - 45, XP025501025, ISSN: 0168-3659, [retrieved on 19931001], DOI: 10.1016/0168-3659(93)90055-A
- RAM R. PATLOLLA ET AL: "Formulation, characterization and pulmonary deposition of nebulized celecoxib encapsulated nanostructured lipid carriers", JOURNAL OF CONTROLLED RELEASE, vol. 144, no. 2, 1 June 2010 (2010-06-01), NL, pages 233 - 241, XP055377516, ISSN: 0168-3659, DOI: 10.1016/j.jconrel.2010.02.006
- JAAFAR-MAALEJ CHIRAZ ET AL: "Beclomethasone-loaded lipidic nanocarriers for pulmonary drug delivery: preparation, characterization and in vitro drug release", JOURNAL OF NANOSCIENCE AND NANOTECHNOLOGY, AMERICAN SCIENTIFIC PUBLISHERS, US, vol. 11, no. 3, 28 February 2011 (2011-02-28), pages 1841 - 1851, XP009516726, ISSN: 1533-4880, DOI: 10.1166/JNN.2011.3119

## Beschreibung

Die Erfindung betrifft die Verwendung von lipophilen Transportpartikeln für kosmetische oder pharmazeutische Wirkstoffe bei der Herstellung pulmonal applizierbarer Inhalativa, wobei die Transportpartikel als Hauptbestandteil Polyglycerolfettsäureester aufweisen und aufgrund der Abwesenheit polymorpher Transformationen auch bei längerer Lagerung weder Volumenveränderungen aufweisen noch das Problem einer Vertreibung des anhaftenden oder eingeschlossenen Wirkstoffs aufgrund einer stärkeren Strukturierung des Kristallgitters und einer damit einhergehenden Kompaktierung zu Tage treten lassen, so dass Wirkstoffladungsgrad und Freisetzungsprofil stabil sind.

Inhalativa für die pulmonale Applikation pharmazeutischer Wirkstoffe bieten verschiedene Vorteile. Zum einen können Erkrankungen mit Beteiligung der Atemwege gezielt mit ihnen behandelt werden, zum anderen können Wirkstoffe, die eine Magen-Darm-Passage nicht unbeschadet überstehen würden und deshalb zumeist intravenös verabreicht werden, bei entsprechend kleinen Abmessungen der arzneistofftransportierenden Partikel mit vergleichbarer Effektivität pulmonal appliziert werden mit dem Vorteil einer gesteigerten Therapietreue seitens der zu behandelnden Patienten. Um einen pharmazeutischen Wirkstoff effektiv pulmonal applizieren zu können, sollte er vorzugsweise in Form von Partikeln inhaliert werden, deren massenbezogen medianer aerodynamischer Durchmesser zwischen 1 µm und 5 µm liegt. Das entspricht zwar nicht der Größe, die für einen Transport tief in die Lunge und die Alveolen zu erwarten wäre, jedoch besteht bei kleineren Partikeln das Problem, dass sie mit dem Luftstrom beim Ausatmen aus der Lunge heraus befördert werden bevor der pharmazeutische Wirkstoff dissoziieren und absorbiert werden konnte. Größere Partikel können hingegen größtenteils nicht tief genug in die Lunge eindringen.

Für die Bereitstellung von Partikeln, die pharmazeutische Wirkstoffe effektiv in die Lunge transportieren können, besteht grundsätzlich die Möglichkeit hydrophile oder lipophile Partikel zu verwenden. Weitverbreitet ist die Verwendung von Zuckern, wie beispielsweise α-Lactose-monohydrat, Cyclodextrinen, Mannitol, Dextrosemonohydrat, aber auch von Metallstearaten, wie Calcium-, Magnesium- oder Zinkstearat oder von Aminosäuren wie Leucin oder Trileucin (Piyush Mehta, "Imagine the Superiority of Dry Powder Inhalers from Carrier Engineering", Journal of Drug Delivery, Volume 2018, Article ID 5635010, 14.01.2018). Die Anforderungen, die an Transportpartikel gestellt werden, betreffen zahlreiche Aspekte, insbesondere eine hohe Einschlusseffektivität für die zu transportierenden Wirkstoffe, gute Oberflächeneigenschaften, Reproduzierbarkeit, niedrige Toxizität, gute Freisetzungseigenschaften und Desagglomeration am Wirkort oder niedrige Hygroskopizität in Hinblick auf gute Lagerstabilität in der Inhalationsvorrichtung. Bezüglich Hygroskopizität, Lagerstabilität, Reproduzierbarkeit und Aufnahmekapazität bieten lipophile Transportpartikel gute Voraussetzungen, die jedoch mit dem Nachteil einhergehen, dass Lipide oder auch Triglyceride zu polymorpher Transformation neigen und dadurch ihre Oberflächeneigenschaften während der Lagerung verändern können. Ein als "blooming" bekannter Effekt der Volumenzunahme aufgrund polymorpher Transformation darf bei lipophilen Transportpartikeln für die pulmonale Applikation keinesfalls auftreten, da die hervorgerufenen gravierenden Volumenveränderungen den Einsatz in Therapeutika oder Diagnostika verbieten würde. Zusätzlich würde eine polymorphe Transformation zu einer strukturierteren und kompakteren Kristallgitterausbildung führen, die eine Vertreibung des anhaftenden oder eingeschlossenen Wirkstoffs zur Folge hätte und damit die Steuerbarkeit des Wirkstoffladungsgrades und des Freisetzungsprofils negativ beeinflussen würde. Es wäre mit einem unerwünschten sogenannten "burst effect" bei der Freisetzung zu rechnen, bei dem unvorhersehbare Mengen des Wirkstoffs schlagartig freigesetzt würden. Aber auch für andere Einsatzgebiete von mikronisierten Transportpartikeln, wie beispielsweise bei dermalen oder transdermalen Applikationen kosmetischer oder pharmazeutischer Wirkstoffe, würde das Auftreten polymorpher Transformation sämtliche Vorteile solcher lipophiler Transportpartikel zunichte machen. Lipophile Transportpartikel für die Inhalation sind zum Beispiel in Silva et al., Powder Technology 239 (2013), Seiten 183-192 offenbart.

Es stellt sich daher die Aufgabe, lipophile Transportpartikel für kosmetische oder pharmazeutische Wirkstoffe bereit zu stellen, die in stabiler kristalliner Modifikation vorliegen und auch für eine pulmonale Wirkstoffapplikation geeignet sind.

Die Aufgabe kann mittels solcher Transportpartikel gelöst werden, die einen oder mehrere Polyglycerolfettsäureester gemäß Anspruch 1 aufweisen. Die Erfindung bezieht sich daher die Verwendung lipophiler, für pharmazeutische und kosmetische Wirkstoffe geeigneter Transportpartikel bei der Herstellung pulmonal applizierbarer Inhalativa gemäß Anspruch 1. Bevorzugte Ausführungsformen ergeben sich aus den Unteransprüchen 2 bis 18.

Überraschend konnte nun erstmals festgestellt werden, dass Transportpartikel, die gemäß Anspruch 1 als Hauptbestandteil Polyglycerolfettsäureester, abgekürzt PGFEs, aufweisen, zur Bereitstellung morphologisch stabiler, lipophiler, pulmonal applizierbarer Transportpartikel geeignet sind, wenn solche PGFEs Verwendung finden, die jeweils aus einer vollständigen oder partiellen Veresterung eines zwei bis acht Glyceryleinheiten aufweisenden, linearen oder verzweigten Polyglycerols mit einer oder mehreren jeweils von 6 bis zu 22 Kohlenstoffatome aufweisenden Fettsäuren erhältlich sind.

Die einfachsten Polyglycerole, die als Ausgangsstoffe für eine zielführende Veresterung eingesetzt werden können, sind lineare und verzweigte Diglycerole mit der Summenformel C₆O₅H₁₄, die industriell in bekannter Art und Weise synthetisch bereit gestellt werden, beispielsweise indem Glycerol mit 2,3-Epoxy-1-propanol basenkatalysiert unter Ausbildung von Etherbindungen umgesetzt oder basenkatalysiert thermal kondensiert wird, wobei die hauptsächlich Diglycerole enthaltende Fraktion nachträglich separiert werden kann.

Diglycerole können in drei verschiedenen strukturisomeren Formen auftreten, nämlich in der linearen Form, bei der die Etherbrücke zwischen den jeweils ersten Kohlenstoffatomen der beiden eingesetzten Glycerolmoleküle entsteht, in der verzweigten Form, bei die Etherbrücke zwischen dem ersten Kohlenstoffatom des ersten und dem zweiten Kohlenstoffatom des zweiten eingesetzten Glycerolmoleküls entsteht, und in einer nukleodentrimeren Form, bei der die Etherbrücke zwischen den jeweils zweiten Kohlenstoffatomen entsteht. Bei der alkalisch katalysierten Kondensation zweier Glycerolmoleküle entsteht etwa zu 80% die lineare Form und zu etwa 20% die verzweigte Form, während die nukleodentrimere Form nur in sehr geringem Maße gebildet wird.

Ebenso können für die Veresterung mit Fettsäuren Polyglyerole mit mehr als zwei und bis zu acht Glyceryleinheiten eingesetzt werden. Allgemein werden die Polyglycerole mit "PG" abgekürzt und mit einer tiefergestellten natürlichen Zahl n versehen, die die Anzahl der Polyglyceryleinheiten angibt, also "PGₙ". Beispielsweise würden Triglycerole als PG₃ angegeben und hätten die Summenformel C₉O₇H₂₀. Die vollständige Veresterung mit einer Fettsäure, zum Beispiel mit Stearinsäure, würde nun an allen freien Hydroxylgruppen des PGₙ-Moleküls stattfinden, im Falle eines linearen PG₃ also am ersten und zweiten Kohlenstoffatom der ersten Glyceryleinheit, am zweiten Kohlenstoffatom der zweiten Glyceryleinheit und am zweiten und dritten Kohlenstoffatom der dritten Glyceryleinheit. Die Summenformel für dieses Beispiel ließe sich daher mit C₉O₇H₁₅R₅ angeben, wobei R jeweils für einen Fettsäurerest stehen würde, im gewählten Beispiel mit der Summenformel C₁₈OH₃₅.

Für die Abkürzung von mit gesättigten unverzweigten Fettsäuren veresterten Polyglycerolen hat sich aber auch die Bezeichnung PG(n)-Cm-Vollester oder gegebenenfalls PG(n)-Cm-Partialester etabliert, wobei das eingeklammerte "n", ähnlich wie bei der Bezeichnung der Polyglycerole, die Anzahl der im Molekül enthaltenen Glyceryleinheiten angibt und m für die Anzahl der Kohlenstoffatome der für die Veresterungsreaktion verwendeten gesättigten Fettsäure steht. Das n steht also für die Anzahl an Glyceryleinheiten mit der Summenformel C₃O₂H₅R bzw. C₃O₃H₅R₂ für randständige Glyceryleinheiten, wobei R für einen Fettsäurerest oder das Wasserstoffatom einer freien Hydroxygruppe stehen kann. PG(2)-C18-Vollester würde somit Polyglycerolfettsäurevollester mit der Summenformel C₇₈O₉H₁₅₀ des Hauptbestandteils bezeichnen. Im Falle der PG-Partialester wird die Anzahl der Fettsäurereste gemittelt, wobei die Summenformel zugleich die Fraktion mit den am häufigsten vorhandenen Veresterungsvarianten angibt. Eine genauere Bezeichnung für die Polyglycerolfettsäurepartialester ergibt sich durch die zusätzliche Angabe der Hydroxylzahl, die ein Maß für den Gehalt an unveresterten Hydroxygruppen ist und damit Auskunft über den Veresterungsgrad des Partialesters gibt. Die Veresterungsreaktionen verlaufen dabei vorzugsweise, vermutlich aus sterischen Gründen, von außen nach innen. Es werden also zunächst die Hydroxygruppen verestert, die dem Fettsäurerest die höchsten Freiheitsgrade erlauben. Die erste Veresterungsreaktion an einem linearen Polyglycerol findet demnach vorzugsweise an der Hydroxygruppe des ersten Kohlenstoffatoms einer randständigen Polyglyceryleinheit statt, die zweite Veresterungsreaktion dann an der Hydroxygruppe des dritten Kohlenstoffatoms der anderendseitig randständigen Polyglyceryleinheit. Im Weiteren werden die Hydroxygruppen an bereits veresterten Positionen unmittelbar benachbarten Kohlenstoffpositionen verestert und so fort.

Unter Fettsäuren werden hier aliphatische Monocarbonsäuren mit 6 bis 22 Kohlenstoffatomen verstanden, die vorzugsweise unverzweigt und gesättigt sind und eine gerade Anzahl Kohlenstoffatome aufweisen, jedoch auch ungeradzahlig, verzweigt und/oder ungesättigt sein können. Bevorzugt werden für die Veresterung zu den als Hauptbestandteil der Transportpartikel verwendeten PGFEs Fettsäuren verwendet, die gesättigt und/oder unverzweigt sind. Weiterhin ist es von Vorteil, für die Veresterung unverzweigte, gesättigte Fettsäuren mit 16, 18, 20 oder 22 C-Atomen einzusetzen, also Palmitin-, Stearin-, Arachin- oder Behensäure.

Vorteilhaft finden solche PGFEs Verwendung, die bei einer Untersuchung des oder der einzelnen PGFEs mittels dynamischer Differenzkalometrie für den Wärmefluss im Zuge der Untersuchung bei dem Aufwärmen jeweils lediglich ein endothermes Minimum und bei dem Abkühlen jeweils nur ein exothermes Maximum aufweisen, da längere Lagerzeiten, erhöhte Temperaturen oder Energieeintrag durch Scherkräfte bei der Applikation auftreten können, die bei ungeeigneten Bestandteilen der Transportpartikel zu deren polymorpher Transformation und schwer steuerbaren Eigenschaften eines entsprechenden Inhalativums führen können. Zusätzliche polymorphe Formen würden sich bei einer Untersuchung mittels dynamischer Differenzkalorimetrie durch das Auftreten eines lokalen exothermen Maximums bei der Erwärmung der Probe sowie eines lokalen endothermen Minimums bei dem Abkühlen der Probe bemerkbar machen. Das erst nach einiger Zeit der Lagerung auftretende "blooming", bei dem aufgrund eines Polymorphismus' eines Bestandteils starke Volumenzunahme auftritt, die makroskopisch sichtbar ist, kann durch Transportpartikelbestandteile, die keinen Polymorphismus aufweisen, vermieden werden. Insbesondere Triglyceride, wie Glyceroltripalmitat oder Glyceroltristearat, können Polymorphismen aufweisen, also jeweils sowohl in einer kristallinen, instabilen α-Modifikation als auch in einer metastabilen β'-Modifikation oder einer stabilen β-Modifikation vorliegen und von einer in die andere Modifikation übergehen. Die Modifikationen unterscheiden sich dabei insbesondere durch die Dicke lamellar gepackter, kristalliner Untereinheiten, die auch als subzelluläre Einheiten bezeichnet werden. Für die α-Modifikation von Glyceroltristearat konnte unter bestimmten Bedingungen beispielsweise eine Schichtung von im Mittel je 6 lamellaren Strukturen pro subzellulärerer Einheit bestimmt werden, nach vollständiger Überführung in die β-Modifikation dann eine Schichtung von im Mittel 10,5 lamellaren Strukturen pro subzellulärer Einheit und eine Zunahme der Kristalldicke um etwa 67 %. Dass dabei die rechnerisch zu erwartende Zunahme um 75 % nicht erreicht wird, ist vermutlich dem Umstand geschuldet, dass die Einzellamellen der β-Modifikation aufgrund einer gegenüber der α-Modifikation auftretenden Schrägstellung eine dichtere lamellare Packung aufweisen (siehe D. G. Lopes, K. Becker, M. Stehr, D. Lochmann et al. in Journal of Pharmaceutical Sciences 104: 4257-4265, 2015). Eine solche dichtere lamellare Packung kann dann zu der bereits erörterten, unerwünschten Vertreibung des anhaftenden oder eingeschlossenen Wirkstoffs führen.

Da die Transportpartikelbestandteile im Endprodukt vorliegen, ist es weiterhin vorteilhaft, wenn die verwendeten PGFEs unterhalb ihrer Erstarrungstemperatur eine stabile subzelluläre Form aufweisen mit bei 40°C und 75% relativer Feuchte über wenigstens 6 Monate, also unter den Lagerungsbedingungen eines beschleunigten Haltbarkeitstests, im Wesentlichen konstanter Dicke der lamellar strukturierten Kristallite gemäß mittels Scherrer-Formel ausgewerteter Kleinwinkel-Röntgenstreuung, englisch "small angle X-ray scattering", abgekürzt SAXS. SAXS erlaubt Rückschlüsse auf die Größe, die Form und die innere Oberfläche von Kristalliten. Die Dicke der jeweiligen Kristallite lässt sich dabei mittels der Scherrer-Formel berechnen, wonach D = Kλ / FWHM cos(θ) gilt. Dabei bezeichnet D die Dicke des Kristalliten und K die dimensionslose sogenannte Scherrer-Konstante, die Aussagen über die Form des Kristalliten erlaubt und im Regelfall in guter Näherung mit 0,9 angenommen werden kann. FWHM steht für englisch "full width at half maximum", also für die Breite des Peaks eines Intensitätsmaximums auf halber Höhe gegenüber dem Hintergrund gemessen im Bogenmaß (rad) und θ ist der Bragg-Winkel, also der Einfallswinkel der Strahlung auf die Netzebene. Während eine Probe von mit 10% Polysorbat 65 stabilisiertem Glyceroltripalmitat nach sechsmonatiger Lagerung bei Raumtemperatur eine Kristallitendicke von 31 nm, entsprechend sieben Lamellen, aufweist und sich deren Kristallitendicke nach sechsmonatiger Lagerung bei 40°C mit 52 nm, entsprechend 12 Lamellen, nahezu verdoppelt, zeigen die vorgeschlagenen Polyglycerolfettsäurepartialester zumeist Kristallitendicken von 20 bis 30 nm, entsprechend 2 bis 4 Lamellen und sind nach sechsmonatiger Lagerung bei 40°C in unveränderter Modifikation stabil. Polyglycerolvollester zeigen demgegenüber zumeist eine auf einen höheren Organisationsgrad hinweisende, leicht erhöhte Kristallitendicke von 30 bis 40 nm, entsprechend 5 bis 8 Lamellen, und sind gleichfalls unter den Lagerungsbedingungen eines beschleunigten Haltbarkeitstests in unveränderter Modifikation stabil.

Ebenso ist es günstig, wenn bei den verwendeten PGFEs unter den genannten Bedingungen der lamellare Abstand gemäß Auswertung des mittels Weitwinkel-Röntgenstreuung, englisch "wide angle X-ray scattering", abgekürzt "WAXS", ermittelten Bragg-Winkels im Wesentlichen konstant ist. Einzeluntersuchungen der vorgeschlagenen Polyglycerolfettsäureester unterhalb ihrer jeweiligen Erstarrungstemperatur mittels WAXS, zeigen für alle untersuchten Polyglycerolfettsäureester ein Intensitätsmaximum, das auf einen Ablenkungswinkel von jeweils 21,4°, entsprechend etwa 2θ, also dem Zweifachen des Braggwinkels, schließen lässt, aus dem sich ein Abstand der Netzebenen von 415 pm ergibt, der hier mit der lamellaren Packungsdichte der untersuchten Moleküle korreliert. Dieser Abstand kann strukturell der α-Modifikation zugeordnet werden, bei der die jeweiligen lamellaren Strukturen in einem hexagonalen Gitter parallel zueinander angeordnet sind mit Ebenen bildenden, aufeinander gestapelten Molekülen. Andere Modifikationen lassen sich nicht identifizieren. Die Stabilität der identifizierten α-Modifikation wurde sowohl bei Raumtemperatur als auch bei 40°C über jeweils 6 Monate ebenfalls mittels WAXS betrachtet. Auch hierbei zeigte sich jeweils ausschließlich die für die untersuchten Polyglycerolfettsäureester überraschenderweise stabile α-Modifikation.

Für die Bereitstellung der Transportpartikel werden vorzugsweise PGFEs aus der folgenden Gruppe gewählt: PG(2)-C18-Vollester, PG(2)-C22-Partialester mit einer Hydroxylzahl von 15 bis 100, PG(2)-C22-Vollester, PG(3)-C16/C18-Partialester mit einer Hydroxylzahl von 100 bis 200, PG(3)-C22-Partialester mit einer Hydroxylzahl von 100 bis 200, PG(3)-C22-Vollester, PG(4)-C16-Partialester mit einer Hydroxylzahl von 150 bis 250, PG(4)-C16-Vollester, PG(4)-C16/C18-Partialester mit einer Hydroxylzahl von 150 bis 250, PG(4)-C16/C18-Vollester, PG(4)-C18-Partialester mit einer Hydroxylzahl von 100 bis 200, PG(4)-C22-Partialester mit einer Hydroxylzahl von 100 bis 200, PG(6)-C16/C18-Partialester mit einer Hydroxylzahl von 200 bis 300, PG(6)-C16/C18-Vollester, PG(6)-C18-Partialester mit einer Hydroxylzahl von 100 bis 200, wobei in den Polyglycerolfettsäureestern mit zwei aufgrund der Anzahl ihrer Kohlenstoffatome verschiedenen Fettsäureresten, diejenigen mit der geringeren Anzahl zu 35% bis 45%, diejenigen mit der höheren Anzahl, entsprechend komplementär, zu 55% bis 65% vorliegen und die aufgeführten Vollester vorzugsweise eine Hydroxylzahl kleiner 5 aufweisen.

Eine vorteilhafterweise zu berücksichtigende Eigenschaft der PGFEs für lipophile Transportpartikel gemäß Anspruch 1 ist die Hydrophobizität, die mittels einer Bestimmung des Kontaktwinkels ermittelt werden kann. Die Bestimmung der Hydrophobizität erfolgt über die Bestimmung des Kontaktwinkels zwischen dem in festem Aggregatzustand befindlichen PGFE und einem Tropfen gereinigten Wassers. Gemäß der Gleichung nach Young gilt cosθ = (γ_{SV} - γ_{SL}) / γ_{LV}, wobei γ_{SL} die Grenzflächenspannung zwischen dem PGFE und Wasser, γ_{LV} die Oberflächenspannung des Wassertropfens und γ_{SV} die Grenzflächenspannung zwischen dem PGFE und der Umgebungsluft angibt. θ ist der Kontaktwinkel. Je größer also der Kontaktwinkel θ, desto größer auch die Grenzflächenspannung zwischen dem PGFE und dem Wasser und desto höher die Hydrophobizität des untersuchten PGFEs. Die Kontaktwinkel korrelieren für die vorgeschlagenen Polyglycerolfettsäureester auch mit dem in der pharmazeutischen Technologie gebräuchlichen HLB-Wert, der auf einer Skala von 0 bis 20 Auskunft über das Verhältnis lipophiler zu hydrophilen Molekülanteilen gibt, wobei der hydrophile Anteil mit steigendem HLB-Wert zunimmt. Der Kontaktwinkel des für die lipophilen Transportpartikel verwendeten PGFEs unter Lagerungsbedingungen sollte für die Bereitstellung von einen oder mehrere pharmazeutische Wirkstoffe aufweisenden Transportpartikeln nur moderaten Änderungen unterliegen, damit die Stabilität der Freisetzungskinetik des oder der pharmazeutischen Wirkstoffe aus den Transportpartikeln nicht negativ beeinflusst wird. Bevorzugt werden daher von den Polyglycerolfettsäureestern solche als Hauptbestandteil der Transportpartikel verwendet, deren Kontaktwinkel mit Wasser bei 40°C ebenso wie bei 20°C nach 16 Wochen weniger als 10° Abweichung vom Ausgangswert aufweist. Mit 40° vergleichsweise hoch und damit einer wünschenswerten Konstanz der Freisetzungskinetik abträglich wäre beispielsweise die Abweichung des Kontaktwinkels unter den benannten Bedingungen für Glyceroltristearin mit Wasser, die auf eine Umlagerung während der Lagerung von der α- in die β-Modifikation zurückzuführen ist. Die Erstarrungstemperatur der für die Transportpartikel verwendeten PGFEs liegt vorzugsweise unter 75°C, jedoch oberhalb von 40°C. Die Erstarrungstemperatur ist hier als der Temperaturwert definiert, an dem während einer Probenanalyse mittels dynamischer Differenzkalorimetrie bei dem Abkühlen das Maximum des höchsten exothermen Peaks des Wärmeflusses auftritt.

Synthesebedingt handelt es sich bei PGFEs stets um Mischungen unterschiedlicher Moleküle, insbesondere im Falle der Partialester. Es ist aber ebenfalls möglich für die Bereitstellung geeigneter Transportpartikel nach Anspruch 1 postsynthetisch solche PGFEs zu mischen, die aus jeweils aufgrund der eingesetzten Reaktionspartner oder der Reaktionsbedingungen voneinander verschiedenen Veresterungsreaktionen erhältlich sind.

Unter dem Begriff Wirkstoff soll hier ein pharmazeutischer oder kosmetischer Wirkstoff verstanden werden. Unter einem pharmazeutischen Wirkstoff wird hier ein Stoff verstanden, der als pharmakologisch aktiver Bestandteil eines Arzneimittels verwendbar ist. Stoffe sind hier chemische Elemente und chemische Verbindungen sowie deren natürlich vorkommende Gemische und Lösungen, Pflanzen, Pflanzenteile, Pflanzenbestandteile, Algen, Pilze und Flechten in bearbeitetem oder unbearbeitetem Zustand, Tierkörper, auch lebender Tiere, sowie Körperteile, Körperbestandteile und Stoffwechselprodukte von Mensch oder Tier in bearbeitetem oder unbearbeitetem Zustand, Mikroorganismen einschließlich Viren sowie deren Bestandteile oder Stoffwechselprodukte. Arzneimittel sind hier Stoffe oder Zubereitungen aus Stoffen, die zur Anwendung im oder am menschlichen oder tierischen Körper bestimmt sind und als Mittel mit Eigenschaften zur Heilung oder Linderung oder zur Verhütung menschlicher oder tierischer Krankheiten oder krankhafter Beschwerden bestimmt sind oder die im oder am menschlichen oder tierischen Körper angewendet oder einem Menschen oder einem Tier verabreicht werden können, um entweder die physiologischen Funktionen durch eine pharmakologische, immunologische oder metabolische Wirkung wiederherzustellen, zu korrigieren oder zu beeinflussen oder eine medizinische Diagnose zu erstellen. Ebenfalls als Arzneimittel gelten hier Gegenstände, die ein Arzneimittel im obigen Sinne enthalten oder auf die ein Arzneimittel im obigen Sinne aufgebracht ist und die dazu bestimmt sind, dauernd oder vorübergehend mit dem menschlichen oder tierischen Körper in Berührung gebracht zu werden, sowie Stoffe und Zubereitungen aus Stoffen, die, auch im Zusammenwirken mit anderen Stoffen oder Zubereitungen aus Stoffen, dazu bestimmt sind, ohne am oder im tierischen Körper angewendet zu werden, die Beschaffenheit, den Zustand oder die Funktion des tierischen Körpers erkennen zu lassen oder der Erkennung von Krankheitserregern bei Tieren zu dienen. Ein kosmetischer Wirkstoff ist ein Stoff oder ein Stoffgemisch, dem bei dermaler Applikation eine gesundheitsfördernde oder -erhaltende Wirkung für die Haut oder Hautanhangsorgane wie Haare und Nägel zugesprochen werden kann, wie beispielsweise Hyaluronsäure, Kollagen, Dexpanthenol, Aloe-Vera-Extrakt, Allantoin oder Bisabolol, für den eine pharmakologische Wirkung jedoch nicht zwingend wissenschaftlich belegt sein muss.

Ihren Zweck erfüllen die bereit gestellten Transportpartikel dann, wenn sie mit einem Wirkstoff, insbesondere einem pharmazeutischen Wirkstoff, beladen werden. Dies geschieht beispielsweise mittels eines Sprühtrocknungsverfahrens gemäß Anspruch 4, bei dem der Wirkstoff und der oder die PGFEs zunächst in einem geeigneten organischen Lösungsmittel gelöst und/oder suspendiert werden und dann mittels Sprühtocknung wieder von dem Lösungsmittel getrennt werden. Als Lösungsmittel geeignet sind beispielsweise der Ether Tetrahydrofuran, der Alkohol Ethanol, das Keton Aceton, der Ester Ethylacetat oder das Alkan Heptan. Es hat sich gezeigt, dass mittels Sprühtrocknung eine Beladung mit einem pharmazeutischen Wirkstoff von bis zu 30 Gewichtsprozent möglich ist.

Ein weiteres geeignetes Herstellungsverfahren für die Transportpartikel der vorliegenden Erfindung, in dessen Rahmen die Transportpartikel mit einem pharmazeutischen Wirkstoff beladen werden, ist die Hochdruckhomogenisation, bei der gemäß Anspruch 6 oder 7 zunächst eine Mischung des PGFEs oder eines PGFE-Gemischs mit Wasser durch Rühren bei einer Temperatur oberhalb der Schmelztemperatur des PGFEs oder des PGFE-Gemischs hergestellt wird, dem der oder die gewünschten pharmazeutischen Wirkstoffe und erforderlichenfalls zusätzlich ein nicht-ionisches Tensid zugesetzt wird. Die Mischung wird dann unter hohem Druck von 100 bis 2000 bar durch eine Homogenisierungsdüse gepresst, aufgefangen und in der Regel mehrfach dieser Prozedur unterzogen bis die gewünschte Tröpfchengröße der Lipidphase einer O/W-Emulsion erreicht ist, um dann durch Abkühlung in eine Suspension von wirkstoffbeladenen Transportpartikeln in Wasser überführt zu werden.

Die PGFEs, die für die Herstellung der lipophilen Transportpartikel verwendet werden, sollten zumindest bei Temperaturen von 40°C oder weniger in festem Aggregatzustand vorliegen, damit die entstehenden Transportpartikel bei gleichen Temperaturen in fester Form vorliegen können. Bei der Beladung mit pharmazeutischen Wirkstoffen ist daher zu beachten, dass ein eutektisches Gemisch mit dem oder den pharmazeutischen Wirkstoffen entstehen kann, dass dann ebenfalls bei 40°C oder weniger in festem Zustand vorliegen sollte. Gleiches gilt für den je nach Wirkstoff erforderlichen Zusatz von Emulgatoren wie nicht-ionischen Tensiden.

Für die pulmonale Applikation von pharmazeutischen Wirkstoffen hat es sich als vorteilhaft erwiesen, wenn die Transportpartikel für die Wirkstoffe einen massenbezogen medianen aerodynamischen Durchmesser (MMAD) von 0,5 µm bis 5 µm aufweisen. Die Transportpartikel im Sinne der vorliegenden Erfindung weisen daher diese MMAD auf. Die Bestimmung des theoretischen MMAD kann dabei rechnerisch aus der Stampfdichte, dem geometrischen Durchmesser, ermittelt mittels Lichtbeugung, und dem Formfaktor, ermittelt mittels Polarisationsmikroskopie, erfolgen. Es hat sich gezeigt, dass aus den PGFEs gemäß Anspruch 1 und insbesondere den PGFEs gemäß Anspruch 13 Transportpartikel mit MMADs von 0,5 µm und 5 µm herstellbar sind, insbesondere wenn die Stampfdichte kleiner 0,4 g/cm³ beträgt.

Für die Lagerungseigenschaften der Transportpartikel ist es vorteilhaft, wenn diese einen Wassergehalt kleiner 2,5% bestimmt mittels Karl-Fischer-Titration aufweisen.

Für die Beladung der Transportpartikel mit Glucosteroiden, wie beispielsweise Dexamethason, ist es von Vorteil, wenn die Transportpartikel neben Polyglycerolfettsäureester auch einen Emulgator, vorzugsweise ein nicht-ionisches Tensid, aufweisen. Insbesondere die Kombination von PG(2)-C18-Vollester mit Poloxamer 188 führt zu einer guten Einschlusseffektivität für Dexamethason.

Je nach Wirkstoff, mit dem die Transportpartikel beladen werden sollen, kann es außerdem von Vorteil für deren Einschlusseffektivität sein, wenn den PGFEs flüssige Lipide beigemischt werden, wobei die Beimischung jedoch so gering zu halten ist, dass der feste Aggregatzustand der Transportpartikel gewährleistet ist. Auch im Bereich der kosmetischen Wirkstoffe kann es sinnvoll sein, diese zunächst in flüssigen Lipiden zu lösen, wie beispielsweise fettlöslich Vitamine.

Lipophile Transportpartikel gemäß Anspruch 1 können vorteilhaft mit pharmazeutischen Wirkstoffen aus der Gruppe der nicht-steroidalen Antirheumatika beladen werden. Hierbei hat sich die Herstellung gemäß Sprüchtrocknungsverfahren nach Anspruch 4 bewährt, wodurch eine Beladung der Transportpartikel von bis zu 30 Gewichtsprozent mit dem Wirkstoff Ibuprofen erreicht werden konnte.

Eine Beladung mit pharmazeutischen Wirkstoffen aus der Gruppe der Glucosteroide ist hingegen vorteilhafter mittels des Hochdruckhomogenisationsverfahrens nach Anspruch 6 oder 7 vorzunehmen. Für Dexamethason konnte beispielsweise eine Einschlusseffektivität der Transportpartikeln in wässriger Suspension von mehr als 90 Gewichtsprozent erreicht werden.

Für die pulmonale Applikation der Transportpartikel, einerlei ob mit oder ohne Wirkstoff beladen, stehen Zerstäuber für in einer Trägerflüssigkeit suspendierte Transportpartikel zur Verfügung, ebenso wie Dosieraerosolvernebler, bei denen die Transportpartikel in einem Treibgas gelöst und/oder suspendiert sind und auf Knopfdruck portioniert freigesetzt werden oder Pulverinhalatoren, bei denen die Transportpartikel portioniert präsentiert werden, um mit dem Luftstrom beim Einatmen inhaliert zu werden. Einerlei mittels welcher Vorrichtung die Inhalation der Transportpartikel ermöglicht wird, seien hier alle Varianten, die eine solche Vorrichtung und die Transportpartikel umfassen unter dem Begriff Inhalativum subsumiert.

Nachfolgend wird die Erfindung anhand von Beispielen und Abbildungen näher erläutert ohne auf diese beschränkt zu sein.

### Beispiel 1

### Bereitstellung mittels Sprühtrocknung mikronisierter, lipophiler, mit Ibuprofen beladener Transportpartikel für einen Pulverinhalator:

Eine Lösung von 1,08 g PG(3)-C22-Partialester-[137], wobei die in eckigen Klammern angehängte Zahl die Hydroxylzahl angibt, und 0,46 g Ibuprofen wird präpariert durch Lösen der Bestandteile in 60 g Tetrahydrofuran, um später einen Gehalt von 2,5 Gewichtsprozent Feststoffanteil zu erreichen. Die Lösung wird in einem Sprühtrockner Procept 4M8-Trix in geschlossener Ringkonfiguration in Stickstoff als Inertgas gesprüht. Dabei werden eine Trocknungssäule als Trocknungsraum und ein kleiner Zyklonabscheider mit einer Druckdifferenz von 60 mbar zum Einsatz gebracht. Die Einlasstemperatur wird auf wenigstens 5°C über dem Siedepunkt des Lösungsmittels eingestellt, die Luftstromgeschwindigkeit auf 0,3 m³/min. Die Lösung wird mit einer Rate von 3,5 g/min durch eine 0,2mm Bi-Fluid-Düse mit einem Düsendruck von 0,9 bar gepresst, entsprechend 3 l/min. Die abgeschiedenen, mit Ibuprofen beladenen Transportpartikel werden aus der Sprühtrocknungsanlage entfernt und für 10 Stunden unter Vakuum aufbewahrt, um Lösungsmittelreste zu entfernen. Es entstehen zu 30 Gewichtsprozent mit Ibuprofen beladene Transportpartikel mit einem theoretischen MMAD von 4,10 µm. Die Schüttdichte beträgt 0,215 g/cm³, die Stampfdichte 0,342 g/cm³, die wahre Dichte 1,069 g/cm³, der Wassergehalt 0,45 %. Die dynamische Differenzkalometrie für das eutektische Gemisch aus Ibuprofen und PG(3)-C22-Partialester-[137] ergibt die nachfolgend gelisteten Werte.
1) unmittelbar nach Bereitstellung der mit Ibuprofen beladenen Transportpartikel;
2) nach einmonatiger Lagerung bei 20°C;
3) nach einmonatiger Lagerung bei 40°C:

| | ad 1) | ad 2) | ad 3) |
|---|---|---|---|
| Start des Schmelzvorgangs: | 58,0°C (± 0,06°C) | 58,1°C (± 0,07°C) | 58,1°C (± 0,07°C) |
| Schmelztemperatur: | 60,9°C (± 0,12°C) | 60,8°C (± 0,07°C) | 60,8°C (± 0,14°C) |
| Kristallisationstemperatur: | 57,9°C (± 0,06°C) | 58,1°C (± 0,07°C) | 58,0°C (± 0,00°C) |
| Schmelzwärme: | 139,2°C (± 5,1°C) | 139,2°C (± 6,2°C) | 139,8°C (± 5,4°C). |

### Beispiel 2

### Bereitstellung mittels Hochdruckhomogenisierung mikronisierter, lipophiler, mit Dexamethason beladener Transportpartikel als Suspension für einen Vernebler

Zu bei 70°C aufgeschmolzenem PG(2)-C18-Vollester werden 0,1 Gewichtsprozent Dexamethason zugesetzt und bei 750 Umdrehungen pro Minute mit einem Magnetrührer für 60 Minuten zu einer klaren Lösung verrührt. 100 ml Präemulsion für die Hochdruckhomogenisation werden hergestellt bei 70°C durch zweiminütiges Mixen von 10 Gewichtsprozent der klaren Lösung, 2,5 Gewichtsprozent Poloxamer 188 und 87,5 Gewichtsprozent gereinigten Wasser mit einem Ultraturrax T25 (Janke & Kunkel), IKA, Deutschland) bei 20.500 Umdrehungen pro Minute hergestellt. Die Präemulsion wird in drei aufeinander folgenden Durchgängen einer Hochdruckhomogenisation unterworfen, die ebenfalls bei 70°C erfolgt. Dabei kommt ein Hochdruckkolbenhomogenisator mit Spaltventil Panda K2 NS1001L (GEA NiroSoavi, Deutschland) zum Einsatz. Die Partikelgröße wird mit einem Mastersizer 2000 (Malvern, Vereinigtes Königreich) bestimmt, bei dem es sich um ein Gerät zur Analyse der Laserstrahlbeugung auf Basis statischer Lichtstreuung handelt. Dazu werden ca. 10 - 30 µl der zu untersuchenden Suspension in die Messzelle gegeben, die bereits 20 ml hochreines Wasser enthält, um eine Trübung zwischen 4% und 6% zu erreichen. Als Teilchenrefraktionsindex wird 1,55, als Teilchenabsorptionsindex 0,01 gesetzt, um bei jeder Messung einen Restwert kleiner 1 zu erhalten. Die Pumpgeschwindigkeit beträgt 1750 Umdrehungen pro Minute. Die Datenanalyse erfolgt aufgrund der Mie-Theorie. Die mediane Partikelgröße (d₅₀) beträgt demnach 212 nm. Die dynamische Differenzkalometrie für die Nanosuspension im Vergleich mit der klaren Lösung aus PG(2)-C18-Vollester und Dexamethason zeigt, dass der Zusatz von Polyoxamer 188 keinen Hinweis auf eine polymorphe Transformation der stabilen Kristallmodifikation des PGFE ergibt.

Nachfolgend werden beispielhaft die Eigenschaften einiger PGFEs anhand von Abbildungen erläutert.

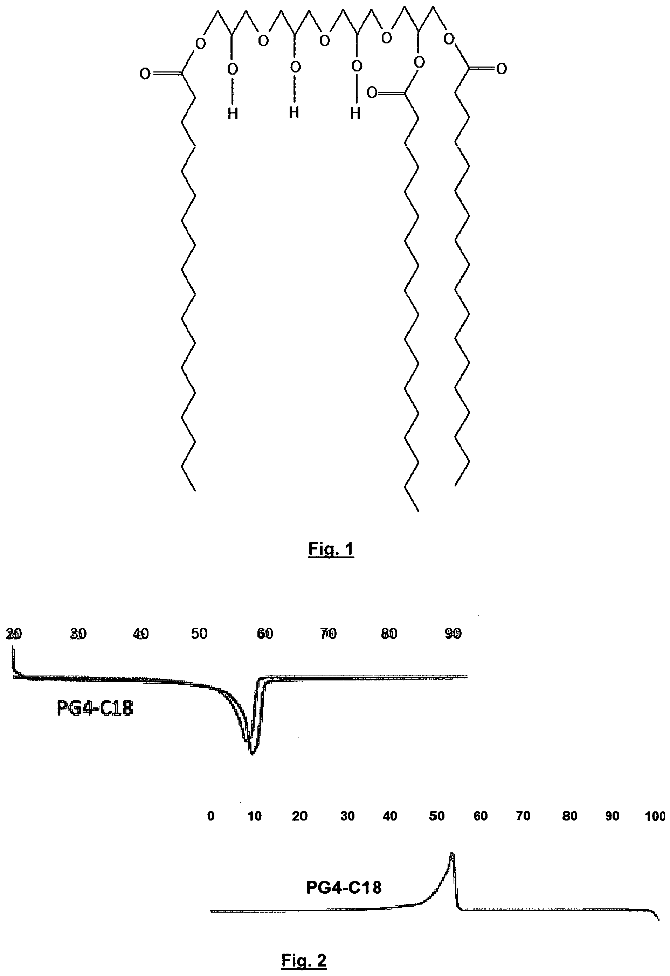

Der Partialester PG(4)-C18 zeigt bei der Untersuchung mittels Gaschromatographie gekoppelt mit Massenspektroskopie (GC-MS) die in **Fig. 1** gezeigte mengenmäßige Hauptstruktur.

**Fig. 2** zeigt die Ergebnisse der Untersuchungen von PG(4)-C18 mittels dynamischer Differenzkalorimetrie, wobei die Temperaturwerte auf der X-Achse des Diagramms dem Wärmefluss in mW/g auf der Y-Achse zugeordnet sind. Das linke Diagramm in **Fig. 2** zeigt zwei nahezu deckungsgleiche Kurven zweier Messungen des Partialesters PG(4)-C18, die jeweils genau ein endothermes Minimum aufweisen, das dem energieverbrauchenden Übergang von der festen in die flüssige Phase beim Schmelzen des Partialesters zugeordnet werden kann. Das rechte Diagramm in **Fig. 2** zeigt für den Partialester PG(4)-C18 genau ein exothermes Maximum, das dem energiefreisetzenden Übergang von der flüssigen in die feste Phase beim Erstarren des Partialesters zugeordnet werden kann. Die Messungen wurden mit einem DSC 204 F1 Phoenix der Nietzsch Gerätebau GmbH, 95100 Selb, Deutschland, durchgeführt. Dabei wurde eine Probe von 3 - 4 mg in einen Aluminiumtiegel eingewogen und bei einer Erwärmungsrate von 5K pro Minute der Wärmefluss kontinuierlich erfasst. Ein zweiter Durchgang wurde mit derselben Erwärmungsrate durchgeführt.

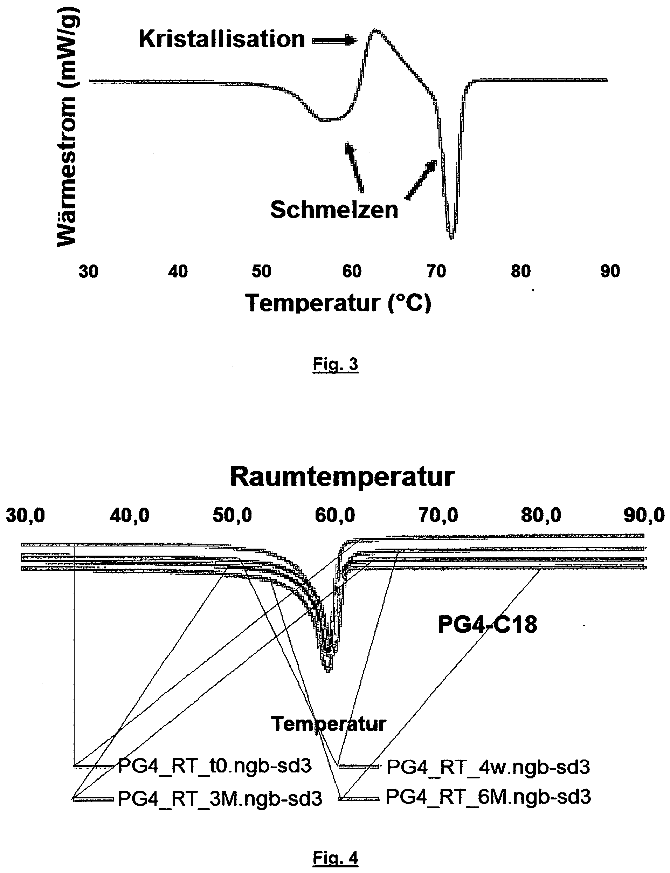

**Fig. 3** zeigt als Kontrast zu dem erwünschten Verhalten der Polyglycerolfettsäureester das typische Verhalten eines polymorphen Triacylglycerols während einer Untersuchung mittels dynamischer Differerenzkalorimetrie beim Aufwärmen. Hier sind zwei lokale endotherme Minima mit einem dazwischen liegenden exothermen Maximum zu erkennen, wobei das erste endotherme, linksseitige Minimum aufgrund des Schmelzens der instabilen α-Modifikation auftritt, gefolgt von dem exothermen Maximum bei der Kristallisation in die stabilere β-Modifikation, die wiederum bei weiterer Temperatursteigerung schmilzt, erkennbar durch das zweite endotherme, rechtsseitige lokale Minimum.

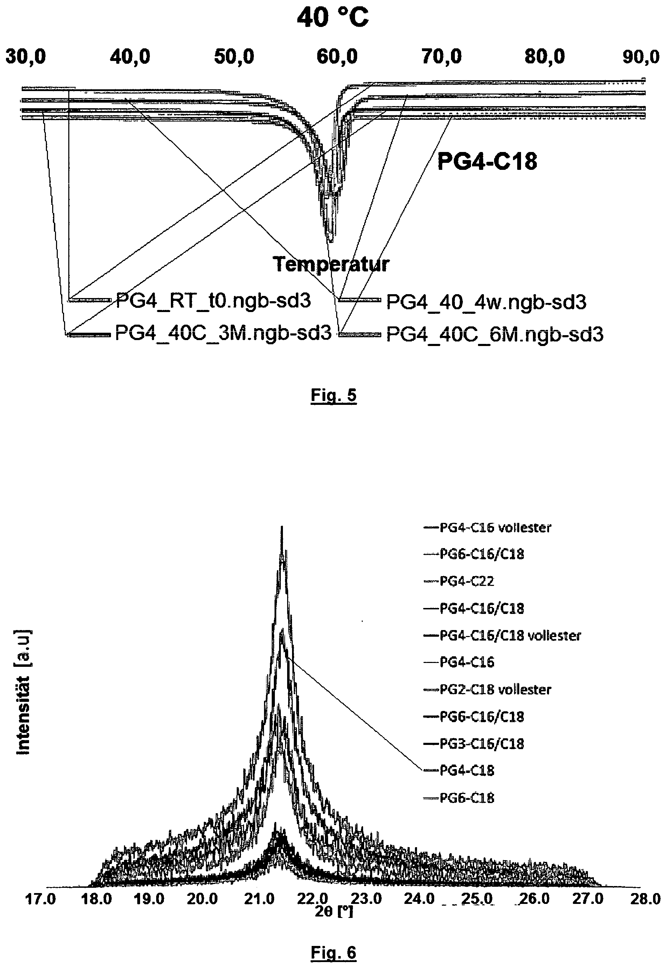

**Fig. 4** zeigt den mittels dynamischer Differenzkalorimetrie untersuchten PG(4)-C18-Partialester beim Aufwärmen nach 6-monatiger Lagerung bei Raumtemperatur. **Fig. 5** zeigt den mittels dynamischer Differenzkalorimetrie untersuchten PG(4)-C18-Partialester beim Aufwärmen nach 6 monatiger Lagerung bei 40°C. In beiden Fällen zeigt sich nach wie vor kein exothermes Maximum, das auf die Kristallisation in eine stabilere Modifikation nach dem Schmelzen hindeuten könnte.

Für die WAXS- und die SAXS-Analysen wurde ein punktfokussierendes Kamerasystem, S3-MICRO, vormals Hecus X-ray Systems Gesmbh, 8020 Graz, Österreich, jetzt Bruker AXS GmbH, 76187 Karlsruhe, Deutschland, ausgestattet mit zwei linearen positionssensitiven Detektoren mit einer Auflösung von 3,3 - 4,9 Angström (WAXS) und 10 - 1500 Angström (SAXS) verwendet. Die Proben wurden in eine Glaskapillare von etwa 2 mm Durchmesser eingebracht, die nachträglich mit Wachs versiegelt und in der Kapillaren-Rotationseinheit platziert. Die Einzelmessungen wurden bei Raumtemperatur für 1300 sec einem Röntgenstrahl mit einer Wellenlänge von 1,542 Angström ausgesetzt.

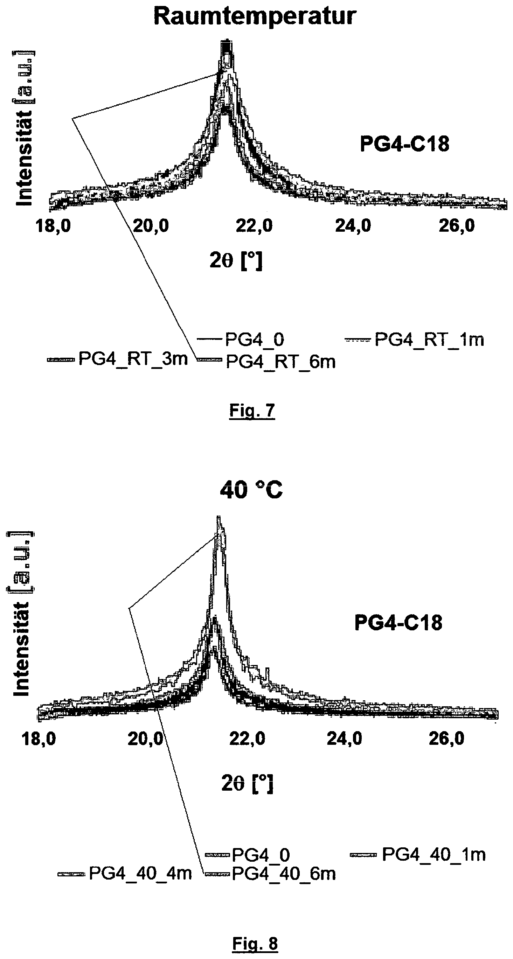

**Fig. 6** zeigt die Ergebnisse der WAXS-Analyse verschiedener Polyglycerolfettsäureester einschließlich PG(4)-C18-Partialester (markiert) unterhalb ihrer Erstarrungstemperatur, die alle ein Intensitätsmaximum bei 2 θ von 21,4° zeigen. Der Bragg-Winkel entspricht einem Abstand der Netzebenen von 415 pm, der typisch ist für die lamellare Packung der α-Modifikation. Das Intensitätsmaximum bleibt sowohl bei Lagerung über 6 Monate bei Raumtemperatur, wie in **Fig. 7** gezeigt, als auch bei Lagerung über 6 Monate bei 40°C stabil, wie in **Fig. 8** gezeigt.

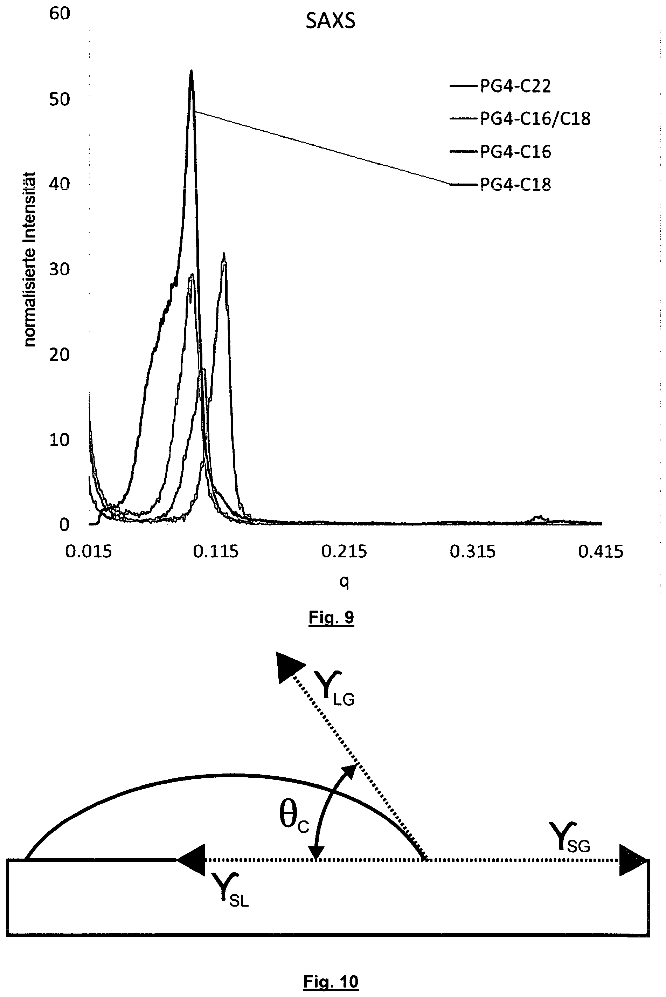

**Fig. 9** zeigt die Ergebnisse der SAXS-Analyse verschiedener Polyglycerolfettsäurepartialester. Für PG(4)-C18-Partialester lässt sich ein lamellarer Abstand von 65,2 Angström ableiten. Die Dicke der Kristalliten beträgt der Scherrer-Formel zufolge 12,5 nm, bei einer Scherrer-Konstanten von 0,9, einer Wellenlänge von 1,542 Angström, einem FWHM-Wert von 0,0111 und einem Bragg-Winkel θ von 0,047 (rad). Die Werte der SASX-Analyse von PG(4)-C18-Partialester blieben auch nach sechsmonatiger Lagerung sowohl bei Raumtemperatur als auch bei 40°C konstant (nicht gezeigt).

Die Auswertung der dynamischen Differenzkalorimetrie erlaubt auch Aussagen über die Erstarrungstemperatur des PG(4)-C18-Partialesters. Der Peak des exothermen Maximums bei dem Abkühlen der Probe erhebt sich zwischen 53,4°C und 57,0°C mit dem Maximum bei 55,2°C, das die Erstarrungstemperatur markiert.

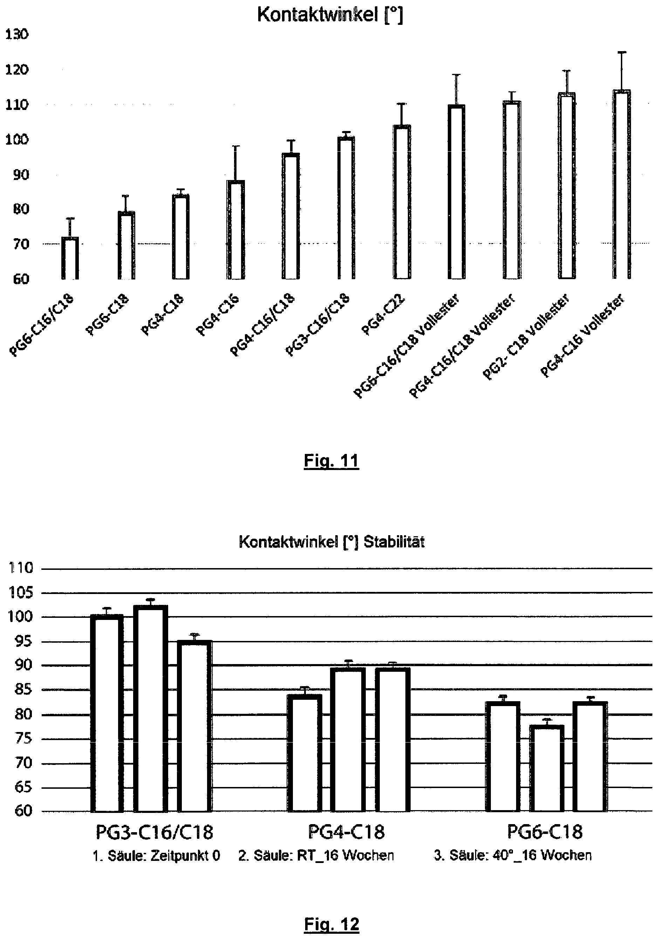

**Fig. 10** zeigt ein Schema, das die Messung des Kontaktwinkels verdeutlicht (vgl. Abs. [0020]). Für PG(4)-C18-Partialester beträgt der Kontaktwinkel etwa 84°, was mit einem HLB-Wert von etwa 5,2 korreliert. Im Vergleich zu weiteren Polyglycerolfettsäureestern ist PG(4)-C18-Partialester den hydrophileren Polyglycerolfettsäureestern zuzuordnen, wie aus **Fig. 11** ersichtlich (dort = PG4-C18). **Fig. 12** zeigt die Veränderung des Kontaktwinkels für PG(4)-C18-Partialester, mittiges Diagramm, gegenüber der Startmessung (linke Säule) nach 16 Wochen bei Raumtemperatur (mittlere Säule) und nach 16 Wochen bei 40°C (rechte Säule). Der Kontaktwinkel ändert sich um nicht mehr als 10°, die Hydrophobizität kann somit im Vergleich mit Monoglycerolfettsäureestern, wie beispielsweise Tristearylglycerol, als stabil bezeichnet werden. Gleiches gilt für die ebenfalls in **Fig. 12** gezeigten PG3-C16/C18-Partialester, linkes Diagramm, und PG6-C18-Partialester, rechtes Diagramm.

## Patentansprüche

1. Verwendung lipophiler, für pharmazeutische und kosmetische Wirkstoffe geeigneter Transportpartikel bei der Herstellung pulmonal applizierbarer Inhalativa,
**dadurch gekennzeichnet,**
**dass** die Transportpartikel einen massenbezogenen medianen aerodynamischen Durchmesser (MMAD) von 0,5 µm bis 5 µm, eine Stampfdichte kleiner 0,4 g/cm³ und als Hauptbestandteil einen oder mehrere Polyglycerolfettsäureester aufweisen, die jeweils erhältlich sind aus einer vollständigen oder partiellen Veresterung eines zwei bis acht Glyceryleinheiten aufweisenden, linearen oder verzweigten Polyglycerols mit einer oder mehreren jeweils von 6 bis zu 22 Kohlenstoffatome aufweisenden Fettsäuren.

2. Verwendung lipophiler Transportpartikel nach Anspruch 1,
**dadurch gekennzeichnet,**
**dass** die Transportpartikel während der Herstellung mit wenigstens einem der Wirkstoffe beladen werden.

3. Verwendung lipophiler Transportpartikel nach Anspruch 2,
**dadurch gekennzeichnet,**
**dass** die Transportpartikel einen Wirkstoffgehalt größer 10 Gewichtsprozent aufweisen.

4. Verwendung lipophiler Transportpartikel nach einem der vorhergehenden Ansprüche,
**dadurch gekennzeichnet,**
**dass** die Transportpartikel nach
i) Lösung und/oder Suspendierung des oder der Polyglycerolfettsäureester zusammen mit einem pharmazeutischen Wirkstoff in einem organischen Lösungsmittel und
ii) anschließender Entfernung des Lösungsmittels mittels Sprühtrocknung wenigstens einen der Wirkstoffe aufweisen.

5. Verwendung lipophiler Transportpartikel nach einem der vorhergehenden Ansprüche,
**dadurch gekennzeichnet,**
**dass** der pharmazeutischer Wirkstoff der Gruppe der nicht-steroidalen Antirhematika (NSAR) zuzuordnen ist.

6. Verwendung lipophiler Transportpartikel nach einem der Ansprüche 1 bis 3,
**dadurch gekennzeichnet,**
**dass** die Transportpartikel nach
i) Herstellung einer Mischung des oder der Polyglycerolfettsäureester mit Wasser und wenigstens einem pharmazeutischen Wirkstoff durch Rühren bei einer Temperatur über der Schmelztemperatur des oder der Polyglycerolfettsäureester,
ii) anschließendem ein- oder mehrfachem Versprühen der Mischung durch eine Hochdruckhomogenisierungsdüse zu einer O/W-Emulsion und
iii) Abkühlung der Lipidphase zu festen Partikeln in Wasser
wenigstens einen der Wirkstoffe aufweisen.

7. Verwendung lipophiler Transportpartikel nach Anspruch 6,
**dadurch gekennzeichnet,**
**dass** der Mischung in Schritt i) zusätzlich wenigstens ein nicht-ionisches Tensid zugesetzt wird.

8. Verwendung lipophiler Transportpartikel nach einem der Ansprüche 1, 2, 3, 6 oder 7,
**dadurch gekennzeichnet,**
**dass** wenigstens ein Wirkstoff der Gruppe der Glucosteroide zuzuordnen ist.

9. Verwendung lipophiler Transportpartikel nach einem der vorhergehenden Ansprüche, **dadurch gekennzeichnet,**
**dass** die Transportpartikel ein oder mehrere inkorporierte flüssige Lipide aufweisen.

10. Verwendung lipophiler Transportpartikel nach einem der vorhergehenden Ansprüche, **dadurch gekennzeichnet,**
**dass** die Fettsäuren für die Synthese der Polyglycerolfettsäureester entweder gesättigt oder unverzweigt oder sowohl gesättigt als auch unverzweigt sind.

11. Verwendung lipophiler Transportpartikel nach einem der vorhergehenden Ansprüche, **dadurch gekennzeichnet,**
**dass** die Fettsäuren für die Synthese der Polyglycerolfettsäureester der Transportpartikel 16, 18, 20 oder 22 Kohlenstoffatome aufweisen.

12. Verwendung lipophiler Transportpartikel nach einem der vorhergehenden Ansprüche, **dadurch gekennzeichnet,**
**dass** eine Untersuchung des oder der einzelnen Polyglycerolfettsäureester mittels dynamischer Differenzkalorimetrie für den Wärmefluss bei dem Aufwärmen jeweils lediglich ein endothermes Minimum und bei dem Abkühlen jeweils lediglich ein exothermes Maxiumum ergibt.

13. Verwendung lipophiler Transportpartikel nach einem der vorhergehenden Ansprüche, **dadurch gekennzeichnet,**
**dass** die Transportpartikel wenigstens einen Polyglycerolfettsäureester aus der folgenden Gruppe aufweisen:
PG(2)-C18-Vollester, PG(2)-C22-Partialester mit einer Hydroxylzahl von 15 bis 100, PG(2)-C22-Vollester, PG(3)-C16/C18-Partialester mit einer Hydroxylzahl von 100 bis 200, PG(3)-C22-Partialester mit einer Hydroxylzahl von 100 bis 200, PG(3)-C22-Vollester, PG(4)-C16-Partialester mit einer Hydroxylzahl von 150 bis 250, PG(4)-C16-Vollester, PG(4)-C16/C18-Partialester mit einer Hydroxylzahl von 150 bis 250, PG(4)-C16/C18-Vollester, PG(4)-C18-Partialester mit einer Hydroxylzahl von 100 bis 200, PG(4)-C22-Partialester mit einer Hydroxylzahl von 100 bis 200, PG(6)-C16/C18-Partialester mit einer Hydroxylzahl von 200 bis 300, PG(6)-C16/C18-Vollester, PG(6)-C18-Partialester mit einer Hydroxylzahl von 100 bis 200, wobei in den Polyglycerolfettsäureestern mit zwei aufgrund der Anzahl ihrer Kohlenstoffatome verschiedenen Fettsäureresten, diejenigen mit der geringeren Anzahl zu 35% bis 45%, diejenigen mit der höheren Anzahl entsprechend komplementär zu 55% bis 65% vorliegen und die aufgeführten Vollester vorzugsweise eine Hydroxylzahl kleiner 5 aufweisen.

14. Verwendung lipophiler Transportpartikel nach einem der vorhergehenden Ansprüche, **dadurch gekennzeichnet,**
**dass** der oder die einzelnen Polyglycerolfettsäureester eine Erstarrungstemperatur zwischen 43°C und 56°C aufweisen.

15. Verwendung lipophiler Transportpartikel nach einem der vorhergehenden Ansprüche, **dadurch gekennzeichnet,**
**dass** die Transportpartikel eine postsynthetische Mischung aus Polyglycerolfettsäureestern aufweisen, die aus jeweils aufgrund der eingesetzten Reakionspartner oder Reaktiongsbedingungen voneinander verschiedenen Veresterungsreaktionen erhältlich sind.

16. Verwendung lipophiler Transportpartikel nach einem der vorhergehenden Ansprüche, **dadurch gekennzeichnet,**
**dass** die Transportpartikel einen Wassergehalt kleiner 2,5% aufweisen.

17. Verwendung lipophiler Transportpartikel nach einem der vorhergehenden Ansprüche, **dadurch gekennzeichnet,**
**dass** die Transportpartikel einen Emulgator aufweisen.

18. Verwendung lipophiler Transportpartikel nach einem der vorhergehenden Ansprüche, **dadurch gekennzeichnet,**
**dass** die Transportpartikel ein nicht-ionisches Tensid aufweisen.

## Claims

1. Use of lipophilic carrier particles which are suitable for pharmaceutical and cosmetic active ingredients in the manufacture of inhalation products for pulmonary application, **characterized in that** the carrier particles have a mass median aerodynamic diameter (MMAD) of 0.5 µm to 5 µm, a tamped density of less than 0.4 g/cm³ and one or more polyglycerol fatty acid esters as the major component, which can respectively be obtained from a complete or partial esterification of a linear or branched polyglycerol containing two to eight glyceryl units with one or more fatty acids respectively containing 6 to 22 carbon atoms.

2. Use of lipophilic carrier particles as claimed in claim 1, **characterized in that** the carrier particles are loaded with at least one of the active ingredients during the manufacture.

3. Use of lipophilic carrier particles as claimed in claim 2, **characterized in that** the carrier particles have an active ingredient content of more than 10% by weight.

4. Use of lipophilic carrier particles as claimed in one of the preceding claims, **characterized in that,** following
i) dissolution and/or suspension of the polyglycerol fatty acid ester or polyglycerol fatty acid esters with a pharmaceutical active ingredient in an organic solvent, and
ii) subsequently, removal of the solvent by means of spray drying, the carrier particles contain at least one of the active ingredients.

5. Use of lipophilic carrier particles as claimed in one of the preceding claims, **characterized in that** the pharmaceutical active ingredient is assigned to the non-steroidal antirheumatic agents (NSAR) group.

6. Use of lipophilic carrier particles as claimed in one of claims 1 to 3, **characterized in that,** following
i) manufacture of a mixture of the polyglycerol fatty acid ester or polyglycerol fatty acid esters with water and at least one pharmaceutical active ingredient by stirring at a temperature which is above the melting temperature of the polyglycerol fatty acid ester or the polyglycerol fatty acid esters,
ii) subsequent spraying of the mixture once or multiple times through a high pressure homogenization nozzle to form an O/W emulsion, and
iii) cooling the lipid phase to form solid particles in water, the carrier particles contain at least one of the active ingredients.

7. Use of lipophilic carrier particles as claimed in claim 6, **characterized in that** in addition, at least one non-ionic surfactant is added to the mixture in step i).

8. Use of lipophilic carrier particles as claimed in one of claims 1, 2, 3, 6 or 7, **characterized in that** at least one active ingredient is assigned to the glucosteroids group.

9. Use of lipophilic carrier particles as claimed in one of the preceding claims, **characterized in that** the carrier particles contain one or more incorporated liquid lipids.

10. Use of lipophilic carrier particles as claimed in one of the preceding claims, **characterized in that** the fatty acids for the synthesis of the polyglycerol fatty acid esters are either saturated or unbranched or both saturated as well as unbranched.

11. Use of lipophilic carrier particles as claimed in one of the preceding claims, **characterized in that** the fatty acids for the synthesis of the polyglycerol fatty acid esters of the carrier particles contain 16, 18, 20 or 22 carbon atoms.

12. Use of lipophilic carrier particles as claimed in one of the preceding claims, **characterized in that** an investigation of the polyglycerol fatty acid ester or individual polyglycerol fatty acid esters by means of heat flux differential scanning calorimetry produces, upon heating up, respectively only one endothermic minimum and upon cooling down, respectively only one exothermic maximum.

13. Use of lipophilic carrier particles as claimed in one of the preceding claims, **characterized in that** the carrier particles contain at least one polyglycerol fatty acid ester from the following group:
PG(2)-C18 full esters, PG(2)-C22 partial esters with a hydroxyl value of 15 to 100, PG(2)-C22 full esters, PG(3)-C16/C18 partial esters with a hydroxyl value of 100 to 200, PG(3)-C22 partial esters with a hydroxyl value of 100 to 200, PG(3)-C22 full esters, PG(4)-C16 partial esters with a hydroxyl value of 150 to 250, PG(4)-C16 full esters, PG(4)-C16/C18 partial esters with a hydroxyl value of 150 to 250, PG(4)-C16/C18 full esters, PG(4)-C18 partial esters with a hydroxyl value of 100 to 200, PG(4)-C22 partial esters with a hydroxyl value of 100 to 200, PG(6)-C16/C18 partial esters with a hydroxyl value of 200 to 300, PG(6)-C16/C18 full esters, PG(6)-C18 partial esters with a hydroxyl value of 100 to 200, wherein, in the polyglycerol fatty acid esters containing two fatty acid residues which are different because of the number of their carbon atoms, those with the smaller number are present in an amount of 35% to 45%, those with the corresponding, complementary larger number are present in an amount of 55% to 65% and the specified full esters preferably have a hydroxyl value of less than 5.

14. Use of lipophilic carrier particles as claimed in one of the preceding claims, **characterized in that** the polyglycerol fatty acid ester or the individual polyglycerol fatty acid esters have a solidification temperature between 43°C and 56°C.

15. Use of lipophilic carrier particles as claimed in one of the preceding claims, **characterized in that** the carrier particles contain a post-synthesis mixture of polyglycerol fatty acid esters which are respectively obtainable from esterification reactions which are different from each other because of the respective reaction partners or reaction conditions which are employed.

16. Use of lipophilic carrier particles as claimed in one of the preceding claims, **characterized in that** the carrier particles have a water content of less than 2.5%.

17. Use of lipophilic carrier particles as claimed in one of the preceding claims, **characterized in that** the carrier particles contain an emulsifier.

18. Use of lipophilic carrier particles as claimed in one of the preceding claims, **characterized in that** the carrier particles contain a non-ionic surfactant,

## Revendications

1. Utilisation de particules de transport lipophiles, appropriées à des principes actifs pharmaceutiques et cosmétiques, dans la fabrication de préparations à inhaler qui sont destinées à une application pulmonaire, **caractérisée en ce que** les particules de transport présentent une médiane massique des diamètres aérodynamiques (MMAD) comprise entre 0,5 µm et 5 µm, une masse volumique tassée inférieure à 0,4 g/cm2, leur constituant principal étant un ou plusieurs esters d'acides gras de polyglycérol dont chacun peut être obtenu en soumettant un polyglycérol linéaire ou ramifié renfermant deux à huit unités de glycéryle à une estérification avec un ou plusieurs acides gras dont chacun renferme 6 à 22 atomes de carbone.

2. Utilisation de particules de transport lipophiles selon la revendication 1, **caractérisée en ce que** les particules de transport sont chargées, pendant la fabrication, avec au moins un desdits principes actifs.

3. Utilisation de particules de transport lipophiles selon la revendication 2, **caractérisée en ce que** la teneur en principe actif des particules de transport est supérieure à 10 pour cent en poids.

4. Utilisation de particules de transport lipophiles selon l'une des revendications précédentes, **caractérisée en ce que** les particules de transport comportant,
i) suite à la mise en solution et/ou suspension du ou des esters d'acide gras de polyglycérol ensemble avec un principe actif pharmaceutique dans un solvant organique,
ii) suivie d'un retrait dudit solvant par atomisation, au moins un desdits principes actifs.

5. Utilisation de particules de transport lipophiles selon l'une des revendications précédentes, **caractérisée en ce que** le principe actif pharmaceutique appartient au groupe des anti-inflammatoires non-stéroïdiens (AINS).

6. Utilisation de particules de transport lipophiles selon l'une des revendications 1 à 3, **caractérisée en ce que** les particules de transport comportent,
i) suite à la réalisation d'un mélange du ou des esters d'acide gras de polyglycérol avec de l'eau et au moins un principe actif pharmaceutique par agitation à une température supérieure à la température de fusion du ou des esters d'acide gras de polyglycérol,
ii) suivi d'une ou de plusieurs pulvérisations du mélange à travers une buse d'homogénéisation à haute pression permettant d'obtenir une émulsion H/E et
iii) d'un refroidissement de la phase lipidique pour obtenir des particules solides dans l'eau, au moins un desdits principes actifs.

7. Utilisation de particules de transport lipophiles selon la revendication 6, **caractérisée en ce que** l'on ajoute, à l'étape i), de manière supplémentaire au moins un tensioactif non-ionique audit mélange.

8. Utilisation de particules de transport lipophiles selon l'une des revendications 1, 2, 3, 6 ou 7, **caractérisée en ce que** au moins un principe actif appartient au groupe des glucostéroïdes.

9. Utilisation de particules de transport lipophiles selon l'une des revendications précédentes, **caractérisée en ce que** les particules de transport comportent un ou plusieurs lipides liquides incorporés.

10. Utilisation de particules de transport lipophiles selon l'une des revendications précédentes, **caractérisée en ce que** les acides gras destinés à la synthèse des esters d'acide gras de polyglycérol sont soit saturés ou non-ramifiés soit à la fois saturés et non-ramifiés.

11. Utilisation de particules de transport lipophiles selon l'une des revendications précédentes, **caractérisée en ce que** les acides gras destinés à la synthèse des esters d'acide gras de polyglycérol des particules de transport renferment 16, 18, 20 ou 22 atomes de carbone.

12. Utilisation de particules de transport lipophiles selon l'une des revendications précédentes, **caractérisée en ce que** lors d'une analyse du et de chacun des esters d'acide gras de polyglycérol par calorimétrie différentielle à balayage afin d'identifier le flux de chaleur, on ne trouve à chaque instance qu'un seul minimum endothermique lors du réchauffement et qu'un seul maximum exothermique lors du refroidissement.

13. Utilisation de particules de transport lipophiles selon l'une des revendications précédentes, **caractérisée en ce que** les particules de transport comportent au moins un ester d'acide gras de polyglycérol issu du groupe suivant :
esters C18 complets de PG(2), esters partiels C22 de PG(2) ayant un indice d'hydroxyle compris entre 15 et 100, esters C22 complets de PG(2), esters C16/C18 partiels de PG(3) ayant un indice d'hydroxyle compris entre 100 et 200, esters C22 partiels de PG(3) ayant un indice d'hydroxyle compris entre 100 et 200, esters C22 complets de PG(3), esters C16 partiels de PG(4) ayant un indice d'hydroxyle compris entre 150 et 250, esters C16 complets de PG(4), esters C16/C18 partiels de PG(4) ayant un indice d'hydroxyle compris entre 150 et 250, esters C16/C18 complets de PG(4), esters C18 partiels de PG(4) ayant un indice d'hydroxyle compris entre 100 et 200, esters C22 partiels de PG(4) ayant un indice d'hydroxyle compris entre 100 et 200, esters C16/C18 partiels de PG(6) ayant un indice d'hydroxyle compris entre 200 et 300, esters C16/C18 complets de PG(6), esters C18 partiels de PG(6) ayant un indice d'hydroxyle compris entre 100 et 200, les esters d'acide gras de polyglycérol dont les radicaux d'acide gras sont différents en ce qui concerne le nombre de leurs atomes de carbone les comportant de manière à ce que ceux avec le nombre plus bas soient présents dans une proportion de 35 % à 45 % et ceux avec le nombre plus élevé en constituent le complément, à savoir une proportion de 55 % à 65 %, et l'indice d'hydroxyle des esters complets mentionnés étant préférentiellement inférieur à 5.

14. Utilisation de particules de transport lipophiles selon l'une des revendications précédentes, **caractérisée en ce que** le ou chacun des esters d'acide gras de polyglycérol ayant une température de solidification comprise entre 43 °C et 56 °C.

15. Utilisation de particules de transport lipophiles selon l'une des revendications précédentes, **caractérisée en ce que** les particules de transport comportent, après la synthèse, un mélange d'esters d'acides gras de polyglycérol qui peuvent être obtenus à partir de réactions d'estérification lesquelles se distinguent les unes des autres en raison des réactifs ou des conditions de réaction mis en œuvre.

16. Utilisation de particules de transport lipophiles selon l'une des revendications précédentes, **caractérisée en ce que** les particules de transport présentent une teneur en eau inférieure à 2,5 %.

17. Utilisation de particules de transport lipophiles selon l'une des revendications précédentes, **caractérisée en ce que** les particules comportent un émulsifiant.

18. Utilisation de particules de transport lipophiles selon l'une des revendications précédentes, **caractérisée en ce que** les particules de transport comportent un tensioactif non-ionique.
